# EUROPÄISCHE PATENTSCHRIFT

(11) **EP 2 373 358 B1**
(45) Veröffentlichungstag und Bekanntmachung des Hinweises auf die Patenterteilung: **27.02.2013**
(21) Anmeldenummer: 09774842.0
(22) Anmeldetag: 11.12.2009
(51) Int. Cl.: A61M 1/16, A61M 1/28

(54) **VORRICHTUNG ZUR BEHANDLUNG EINER MEDIZINISCHEN FLÜSSIGKEIT UND VERFAHREN ZUR ÜBERPRÜFUNG IHRER DICHTIGKEIT**
APPARATUS FOR TREATING A MEDICAL LIQUID, AND METHOD FOR CHECKING THE LEAKTIGHTNESS OF THE APPARATUS
DISPOSITIF DE TRAITEMENT D'UN LIQUIDE MÉDICAL, ET PROCÉDÉ DE VÉRIFICATION DE L'ÉTANCHÉITÉ DE CE DISPOSITIF

(30) Priorität: 12.12.2008 DE 102008062037
(43) Veröffentlichungstag der Anmeldung: 12.10.2011
(73) Patentinhaber: Fresenius Medical Care Deutschland GmbH, 61352 Bad Homburg (DE)
(72) Erfinder: MÜLLER, Ralf, 61348 Bad Homburg (DE); SCHEUNERT, Peter, 61381 Friedrichsdorf (DE); GERLACH, Daniel, 60316 Frankfurt am Main (DE); GÜNTHER, Götz, verstorben (DE)
(74) Vertreter: Laufhütte, Dieter
(86) Internationale Anmeldenummer: PCT/EP2009/008881
(87) Internationale Veröffentlichungsnummer: WO 2010/066441

(56) Entgegenhaltungen:
- DE-A1- 10 224 750
- DE-A1-102007 042 964
- DE-C1- 10 157 924
- US-A1- 2005 126 998
- US-A1- 2009 012 454

## Beschreibung

Die Erfindung betrifft eine Vorrichtung zur Behandlung einer medizinischen Flüssigkeit nach dem Oberbegriff des Anspruchs 1. Des weiteren betrifft die Erfindung ein Verfahren zur Überprüfung der Dichtigkeit der vorgenannten Vorrichtung.

Bei einer entsprechenden Vorrichtung zur Behandlung einer medizinischen Flüssigkeit kann es sich beispielhaft um eine Blutbehandlungsmaschine handeln, wie sie z. B. bei der Hämodialyse oder bei der Peritonealdialyse zum Einsatz kommt. Die medizinische Kassette umfasst bei einem solchen Einsatz die blut- bzw. dialyseflüssigkeitsführenden Kanäle und steht über die Koppelfläche mit Aktoren und Sensoren der Behandlungsmaschine in Verbindung. Die medizinische Kassette kann so als kostengünstiges Einwegteil ausgeführt werden, während die Aktoren zur Steuerung des Flüssigkeitsstroms durch die Kassette sowie die Sensoren, z. B. zur Leveldetektion oder zur Druckmessung, in die Behandlungsmaschine integriert sind.

Solche als Einwegartikel ausgeführten medizinischen Kassetten bestehen dabei aus einem dünnwandigen dreidimensionalen Kunststoffhartteil mit einem ebenen umlaufenden Auflagerand und diversen Vertiefungen (Kammern, Stegen und Kanälen). In den von diesen dreidimensionalen Strukturen des Kunststoffhartteils gebildeten Kammern und Kanälen können nun medizinische Flüssigkeiten wie z. B. Dialysat oder Blut geführt werden. Die Auflageebene der Kassette wird durch eine flexible Folie, vorteilhafterweise einer Polymerfolie, die umlaufend mit dem Auflagerand des Hartteils verbunden, insbesondere verschweißt und/oder verklebt ist, flüssigkeitsdicht verschlossen. Die medizinische Kassette wird im Gebrauch mit der flexiblen Folie an die Ankoppelfläche der Behandlungsmaschine angepresst, so dass Aktoren und Sensoren der Behandlungsmaschine an der Polymerfolie aufliegen. Zusätzlich wird durch diese Anpressung die flexible Folie mit Stegen der Kassette verpresst und sorgt so für eine fluiddichte Trennung der flüssigkeitsführenden Kanäle im Hartteil durch die Stege und die flexible Folie.

Des weiteren können die Kassettenkanäle auch mit der flexiblen Folie verschweißt oder verklebt sein, um eine fluiddichte Trennung der flüssigkeitsführenden Kanäle zu erreichen.

Die Ankoppelfläche der Behandlungsmaschine weist dementsprechend üblicherweise Aktoren, Sensoren und Anpresskraftübertragungsflächen auf. Die Aktoren und Sensoren der Blutbehandlungsmaschine sind dabei im angekoppelten Zustand der Kassette gegenüber den flüssigkeitsführenden Kanälen der Kassette angeordnet. Die Aktoren können hierdurch durch Herabdrücken der Folie Ventile bilden, indem die flexible Folie in Bereiche der flüssigkeitsführenden Kanäle hineingedrückt wird und diese verschließt. Die Sensoren messen beispielsweise Druck oder Temperatur der in den flüssigkeitsführenden Kanälen befindlichen Flüssigkeit. In einer Ausführungsform der Kassette können die Anpresskraftübertragungsflächen die flexible Folie gegen Dichtungsstege des Hartteils pressen, welche die flüssigkeitsführenden Kanäle umgeben, um diese gegeneinander und gegen den Rest der Kassette abzudichten. Alternativ können die Kassettenkanäle auch verschweißt oder verklebt sein. Die Ankoppelfläche wird üblicherweise von einer planen Oberfläche eines Trägerelementes, welche z. B. aus Metall gefertigt ist, in welche Aufnahmen für die Sensoren und die Aktoren vorgesehen sind, und den plan in diese Aufnahmen eingesetzte Sensoren gebildet.

Auf der Ankoppelfläche der Behandlungsmaschine ist üblicherweise eine flexible Matte angeordnet, die beispielsweise aus Silikon oder einem anderen elastomeren Material besteht. Dies hat den Vorteil, dass die Sensoroberflächen vor Umgebungseinflüssen geschützt werden und dass die Maschinenoberfläche flüssigkeitsdicht und somit ideal hygienisch reinigbar ist. Die flexible Matte stellt dabei einen Teil der Behandlungsmaschine dar, an welchen die Kassette als einen Einwegteil angekoppelt wird. Durch die Flexibilität der Matte wird die Funktionalität der Aktoren gewahrt. Zudem kann die flexible Folie über die flexible Matte gut mit der Ankoppelfläche verpresst werden, wodurch ein guter Kontakt mit den Aktoren, Sensoren und Anpresskraftübertragungsflächen ermöglicht wird.

Bei der Ankopplung der Sensoren an die Folienoberfläche besteht bei bekannten Systemen die Schwierigkeit, eine gute Kopplung zu erreichen, um korrekte Messwerte zu erhalten. Speziell Luft, die in der Übertragungsstrecke zwischen der flexiblen Folie und der Sensoroberfläche beim Einlegen der Kassetten eingeschlossen wird, führt zu einer Verfälschung der Messergebnisse. Dies gilt für Drucksensoren (insbesondere bei der Messung von Drücken, die kleiner sind als der Umgebungsdruck), aber auch bei der Leveldetektion und ebenfalls bei Aktoren wie z. B. Ventilen. Bei der Ankopplung sollten deshalb unerwünschte Lufteinschlüsse zwischen der äußeren Oberfläche der flexiblen Folie und der aufliegenden Mattenoberfläche der flexiblen Matte beseitigt werden. Üblicherweise erfolgt dies durch Luftabsaugung.

Aus der DE 101 57 924 C1 und der DE 102 24 750 A1 ist es bereits bekannt, den Lufttransport mittels definiert vorgegebener integrierter Mattenkanäle auf der maschinenseitigen Rückseite der Maschinenmatte zu realisieren. Die Luftleitung von der Oberfläche der flexiblen Folie durch die Matte hindurch zu den maschinenseitig angeordneten Luftkanälen erfolgt dabei lokal durch durchgehende Schlitze im Bereich der Mattenkanäle. Hierdurch erfolgt der Lufttransport jedoch lediglich an genau definierten Stellen der flexiblen Folie der Kassette, an welchen die Luft durch die Schlitze in der Matte zu den maschinenseitig angeordneten Mattenkanälen abgesaugt wird. Diese Mattenkanäle müssen sich deshalb im Bereich der flüssigkeitsführenden Kanäle der Kassette befinden, um dort eine gute Absaugung zu gewährleisten, was zu Sicherheitsproblemen führen kann. Bei dieser Ausführung ist die Sensoroberfläche nicht mehr ideal von Umgebungseinflüssen geschützt und nicht unbedingt hermetisch dicht von der Matte abgeschlossen, so dass sich beispielsweise hygienische Probleme ergeben können.

Zur Verbesserung der zuverlässigen Luftabsaugung und zur sicheren Verhinderung von Luftinseln wird in der DE 10 2007 042 964 A1 eine Vorrichtung zur Behandlung einer medizinischen Flüssigkeit vorgeschlagen, bei der im angekoppelten Zustand der Kassette zwischen der flexiblen Folie und der Ankoppelfläche eine Schicht aus einem luftdurchlässigen porösen Material angeordnet ist, durch welche Luft während des Ankoppelvorganges und/oder bei angekoppelter Kassette flächig absaugbar ist.

Zur Erfüllung der hygienischen Anforderungen ist es notwendig, eine geschlossene, flüssigkeitsdichte Oberfläche ohne Dichtstellen, Fugen oder Materialübergängen herzustellen. Gerade hierzu dient die zuvor erwähnte durchgehende flexible Matte, die beispielsweise aus Silikonkautschuk besteht. Diese trennt Sensoren und Aktoren von der Kassettenfolie. Aufgrund dieser Trennung ergeben sich zwei Bereiche, die zur Ankopplung der Kassette evakuiert werden müssen, nämlich der Bereich zwischen der Matte und der Ankoppelfläche und zum zweiten zwischen der Mattenoberseite und der Kassettenfolie. Während in der vorgenannten DE 10 2007 042 964 A1 die maschinenseitige Absaugung zwischen der Ankoppelfläche und der dieser zugewandten Mattenseite auf die ausreichend vorgesehenen Luftleitkanäle gelöst ist, ist die Ankopplung auf der Kassettenseite aufgrund hoher Anforderungen an die Reinigbarkeit und der Maschinenoberfläche aufwendiger.

Sollte es beispielsweise durch Luftleckagen von Außen zu einem Zwischenraum zwischen der flexiblen Matte und der Kassettenfolie kommen, kann dies zum Abkoppeln der Aktoren und zu Fehlmessungen der Sensoren führen.

Gründe für ein Zusammenbrechen des Vakuums zwischen der flexiblen Matte und der auf dieser aufliegenden Folie der Kassette können eine unzureichende Abdichtung zur Umgebungsluft sein. Bei der Absaugung der Kassettenfolie wird eine gute Luftdurchlässigkeit zwischen der Silikonmatte und der Folie gefordert. Diese muss jedoch im Randbereich der Kassette unterbrochen sein. Hierdurch wird an die Dichtigkeit zwischen der Folie und der flexiblen Matte eine hohe Anforderung gestellt, damit verhältnismäßig hohe Druckdifferenzen aufrechterhalten werden können. Kommt es durch Schmutz, Beschädigungen, Einfallstellen oder Fremdkörper zur Undichtigkeit, kann das Vakuum nicht aufrechterhalten werden.

Zum Absaugen eventuell zwischen der Folie und der flexiblen Matte vorhandenen Luft wird in bekannten Vorrichtungen eine mit einer Hydrophobmembran geschützte Absaugöffnung vorgesehen. Die bekannten Systeme sind jedoch durchaus störanfällig, wobei hier entsprechende Störungen mit bekannten Vorrichtungen nicht detektiert werden können. Kommt es beispielsweise durch Flüssigkeit zum Verschluss der Hydrophobmembran der vorgenannten Absaugöffnung, kann anhand der Maschine nicht festgestellt werden, ob der Zwischenraum zwischen Folie und flexibler Membran tatsächlich evakuiert wurde. Die Absaugung kann ebenfalls durch eine Öffnung in der Matte 12 realisiert werden. Dadurch können maschinenseitige Leckdetektoren auch eine Flüssigkeitsleckage erkennen, auch wenn eine Schutzvorrichtung mit einer Hydrophobmemran vorgesehen wird.

Kommt es andererseits durch Knicken des Pneumatikschlauchs in der Maschine zu einem Verschluss des Schlauchs, wird der Absaugvorgang der Luft aus dem Zwischenraum zwischen Folie und flexibler Matte unterbrochen.

Ein besonders kritischer Fall, der im Extremfall auftreten kann, besteht darin, dass bei einer Folienbeschädigung, beispielsweise Blut aus der Kassette austritt und den Zwischenraum zwischen Folie und flexibler Matte füllt. Hier gilt es nicht nur die Ankopplung der Sensoren und Aktoren an der Kassette zu gewährleisten, sondern auch den Blutverlust und eine potentielle Kontamination des Blutes des Patienten zu vermeiden.

Grundsätzlich lässt sich durch die Evakuierungsmöglichkeiten zwischen der Folie und der flexiblen Matte eine hinreichend gute Absaugung gewährleisten. Hierbei ist nicht nur die Absaugung von Luft, sondern auch diejenige von Flüssigkeiten - wie Blut - möglich. Dadurch kann bei einer Flüssigkeitsleckage genau wie bei einer Gasleckage davon ausgegangen werden, dass das eindringende Medium bis zur Absaugöffnung gefördert wird.

Aufgabe der vorliegenden Erfindung ist es nun, eine gattungsgemäße Vorrichtung derart weiterzubilden, dass in einfacher Weise eine Überwachung der exakten Ankopplung zwischen der aus einer flexiblen Matte bestehenden Ankoppelfläche der Behandlungsmaschine einerseits und der Kassette bzw. der die Kassette abdeckenden Folie andererseits gewährleistet ist.

Erfindungsgemäß siehe Anspruch 1, wird diese Aufgabe durch eine Vorrichtung zur Behandlung der medizinischen Flüssigkeit gelöst, welche eine Behandlungsmaschine mit einer Ankoppelfläche und einer auf der Ankoppelfläche angeordneten flexiblen Matte umfasst, wobei eine Kassette aus einem Hartteil mit flüssigkeitsführenden Kanälen, welche von einer flexiblen Folie abgedeckt sind, über die flexible Matte an die Ankoppelfläche der Behandlungsmaschine ankoppelbar ist. Die zwischen der flexiblen Folie und der flexiblen Matte vorhandene Luft kann über Absaugöffnungen in der Kassette abgesaugt werden. In einer alternativen Ausführungsform kann die Luft auch durch Öffnungen in der Matte evakuiert werden. In einer weiteren Ausführungsform wird die Luft manuell durch Verklemmen der Kassette an die Matte entfernt. Hierbei wird durch mechanischen Druck (beispielsweise durch einen aufsitzenden Hebel oder eine Tür) die Luft zwischen Kassette und Matte herausgepresst. Die flexible Matte kann nun im Bereich oberhalb der Messvorrichtung flexibler ausgeführt werden als in den übrigen Bereichen, um eine dortige Auslenkung durch Druckunterschiede zwischen den Druckniveaus beidseitig der flexiblen Matte zu erleichtern.

Statt eines Sensors, der die Auslenkung bzw. Elongation der Matte überwacht, kann auch ein geeigneter Kraftsensor eingesetzt werden. Bei Einsatz eines entsprechenden Kraftsensors kommt es nur zu vernachlässigbaren Auslenkungen der Matte im Bereich der Messkammer. Der Sensor fängt nämlich die Matte im Bereich des Messraums ab und überwacht die Krafteinwirkung auf die Matte in diesem Bereich.

Besondere Ausgestaltungen der Erfindung ergeben sich aus den sich an den Hauptanspruch anschließenden Unteransprüchen.

Demnach kann die flexible Matte oberhalb der Messeinrichtung dadurch flexibler ausgeführt sein, dass sie dünner als in den übrigen Bereichen ausgeführt ist. Aufgrund der dünneren Ausführung der an sich flexiblen Matte kann gerade der Bereich oberhalb der Messeinrichtung weiter ausgelenkt werden und damit in seiner Auslenkung gut detektiert werden. Hierdurch lässt sich ein eventueller Differenzdruck der benachbarten evakuierten Räume auf einfache und sichere Weise überwachen. Alternativ kann die flexible Matte oberhalb der Messeinrichtung auch durch eine Ringnut umfasst sein, die eine Auslenkung der Matte im Bereich oberhalb der Messeinrichtung ebenfalls erleichtert.

Vorteilhaft erfolgt die Überwachung der Auslenkung des Bereichs der flexiblen Matte oberhalb der Messeinrichtung durch einen Sensor.

Der Sensor kann vorteilhaft ein Taster oder auch ein berührungslos arbeitender Sensor, wie beispielsweise ein Ultraschallsensor, ein kapazitiver Sensor oder auch eine Lichtschranke sein. Der Sensor kann ebenso ein Kraft- oder Drucksensor oder Ähnliches sein. Diese Sensoren bieten den Vorteil, dass zur Detektion der Druckdifferenz zwischen den benachbarten evakuierten Räumen nur vernachlässigbar kleine Auslenkungen der Matte oberhalb der Messeinrichtung notwendig sind.

Ein erfindungsgemäßes Verfahren, siehe Anspruch 14 zur Überprüfung der Dichtigkeit der Vorrichtung nach einem der vorgenannten Ansprüche, nach den Schritten:
- Initiale Überprüfung der Messeinrichtung vor dem Ankoppeln der Kassette durch Evakuieren des Zwischenraums zwischen Matte und Ankoppelfläche,
- Ankoppeln der medizinischen Kassette an die Ankoppelfläche der Behandlungsmaschine über eine auf der Ankoppelfläche angeordnete flexible Matte und
- Absaugen von Luft zwischen der flexiblen Folie und der flexiblen Matte während des Ankoppelvorgangs und/oder bei angekoppelter Kassette besteht darin, dass das zwischen der flexiblen Folie und der Ankoppelfläche gebildete Vakuum durch einen Vergleich des Drucks zwischen der flexiblen Matte und der Folie einerseits mit dem Druck zwischen der flexiblen Matte und der Ankoppelfläche andererseits überprüft wird.

Der erste Schritt der vorgenannten Verfahrensfolge betrifft die initiale Überprüfung der Messeinrichtung. Diese initale Überprüfung ist ein Test der Sensorvorrichtung vor dem Ankoppeln der Kassette. Hier wird die Luft zwischen der Matte und der Ankoppelfläche evakuiert. Dies erfolgt durch eine Absaugöffnung in der Ankoppelfläche. Entsprechende Absaugkanäle sorgen für eine vollfächige Evakuierung der Luft zwischen der Ankoppelfläche und der Matte. Durch den hierdurch entstehenden Druckgradienten zwischen Sensorraum und Außenluft bewegt sich die Matte bei flexibler Ausführung in Richtung zum Sensor, der die Elongation der Matte überwacht. Alternativ bei Verwendung eines Kraftsensors wirkt hier eine Kraft auf den alternativ vorgesehenen Kraftsensor. Während des Selbsttests muss also ein entsprechendes Signal erkannt werden, um die einwandfreie Funktionsweise der Messeinrichtung bestätigen zu können.

Zur Bestimmung des Differenzdruckes wird die Auslenkung der flexiblen Matte oberhalb eines Sensors gemessen.

Weitere Merkmale, Einzelheiten und Vorteile der Erfindung werden anhand eines in der Zeichnung dargestellten Ausführungsbeispiels erläutert. Es zeigen:
- Figur 1:: den prinzipiellen Aufbau einer an einer Behandlungsmaschine angekoppelten Kassette im Teilschnitt gemäß dem Stand der Technik,
- Figur 2:: eine Ausführungsform der erfindungsgemäß ausgebildeten Vorrichtung zur Behandlung der medizinischen Flüssigkeit in einer ersten Arbeitsstellung und
- Figur 3:: die Vorrichtung gemäß Figur 2 in einer zweiten Arbeitsstellung.

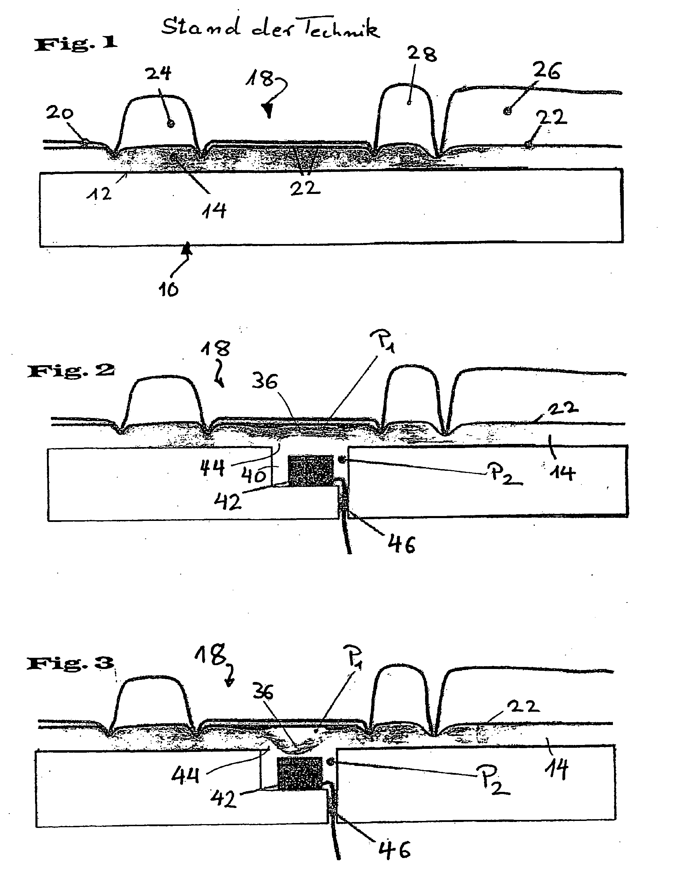

Figur 1 zeigt eine Vorrichtung zur Behandlung einer medizinischen Flüssigkeit, wie sie im Stand der Technik, beispielsweise für die Hämodialyse oder die Peritonealdialyse verwendet wird. Solche Vorrichtungen können aber auch in einer Vielzahl von anderen Einsatzgebieten verwendet werden, bei welchen eine Einwegkassette, die auch als Disposable bezeichnet wird, verwendet wird und über eine Ankoppelfläche mit Sensoren und Aktoren einer Behandlungsmaschine gekoppelt wird.

Die Behandlungsmaschine 10 weist dabei als Ankoppelfläche 12 und eine auf der Ankoppelfläche 12 angeordnete flexible Matte 14 auf. Eine Kassette 18 umfasst ein Hartteil 20 sowie eine flexible Folie 22 über das Hartteil 20 bzw. die flexible Folie 22 werden unterschiedliche Blutkanäle 24, 26, 28 definiert.

Gemäß der in den Figuren 2 und 3 dargestellten beispielhaften Ausführungsform der Erfindung wird nun die Überwachung des Vakuums zwischen der Folie 22 und der flexiblen Matte 14 durch einen Vergleich des maschinenseitigen Drucks mit dem Druck zwischen der flexiblen Matte 14 und der Ankoppelfläche 12 mit dem Druck zwischen der Folie 22 und der flexiblen Matte 14 realisiert. Hierzu ist, wie in den Figuren 2 und 3 dargestellt, der Bereich 36 der flexiblen Matte 14 oberhalb eines Sensors 42 vorzugsweise dünner als in ihren anderen Bereichen ausgeführt und dadurch flexibler und nachgiebiger gestaltet. Nach dem initialen Test ist die Luft zwischen flexibler Matte 14 und Ankoppelfläche 12 evakuiert. Der Teil der flexiblen Matte, der sich oberhalb des Sensors 42 befindet, wird dementsprechend in Richtung des Sensors durch den Aussendruck bewegt. Bei korrekter Ankoppelung der Kassette 18 an die Matte 14 ist die Luft zwischen Kassette 18 und Matte 14 evakuiert. Die Druckdifferenz zwischen den Drücken P1 im Raum zwischen Kassette 18 und Matte 14 und P2 im Raum zwischen Matte 14 und Ankoppelfläche 12 ist demnach Null. Auf das Teilstück 36 der flexiblen Matte oberhalb des Sensors wirken somit keine äußeren Kräfte, und die Matte nimmt in diesem Bereich seine vormalig flache Form an, wie in Figur 2 dargestellt.

In Figur 3 ist der Fall dargestellt, dass die Ankopplung der Kassette 18 an die Matte 14 nicht ordnungsgemäß stattgefunden hat, das heißt, die Luft zwischen Kassette 18 und Matte 14 wurde nicht vollständig entfernt. Der Druck P1 im Raum zwischen Kassette 18 und Matte 14 ist somit größer als der Druck P2 Im Raum zwischen Matte 14 und Ankoppelfläche 12, wodurch das Teilstück 36 der flexiblen Matte oberhalb des Sensors in Richtung des Sensors gewölbt verbleibt.

Der Sensor 42 detektiert in geeigneter Weise die Auslenkung der flexiblen Matte oberhalb des Sensors. Dieser Sensor kann beispielsweise aus einem berührungslosen Sensor, wie einem Ultraschallsensor oder kapazitiven Sensor bestehen. Statt des hier dargestellten Sensors 42 kann auch beispielsweise eine Reflexlichtschranke genutzt werden, die bei Auslenkung der flexiblen Matte 14 im Bereich des flexibler gestalteten Bereichs 36 vollständig abgedeckt wird.

Zur Verbesserung der Auslenkung des dünner ausgestalteten Bereichs 36 der flexiblen Matte 14 ist eine flexible Ringnut 44 vorgesehen, wie in den Figuren 2 und 3 dargestellt.

In einer alternativen Ausführungsform ist das Teilstück 36 der flexiblen Matte 14 in Richtung des Sensors 42 vorgeformt. Hierbei ergibt sich ohne Evakuierung der Luft zwischen flexibler Matte 14 und Ankoppelfläche 12 die Situation wie in Figur 3 dargestellt. Bei korrekter Ankoppelung der Kassette 18 an die Matte 14 ist die Luft zwischen Kassette 18 und Matte evakuiert. Der Druck P1 im Raum zwischen Kassette 18 und Matte 14 ist demnach kleiner als der Druck P2 im Raum zwischen Matte 14 und Ankoppelfläche. Auf das Teilstück 36 der flexiblen Matte oberhalb des Sensors wirkt somit eine Kraft vom Sensor 42 weg, wodurch das Teilstück 36 der flexiblen Matte 14 gegen die Kassette 18 gedrückt wird, wie in Figur 2 dargestellt. Auch bei dieser Ausführungsform wird das ordnungsgemäße Ankoppeln der Kassette 18 an die Matte 14 durch Messung der Auslenkung des Teilstücks 36 der flexiblen Matte 14 durch einen geeigneten Sensor überwacht.

Wird nun, wie im Beispiel der Figur 3 dargestellt, festgestellt, dass das Vakuum zwischen der Folie 22 und der flexiblen Matte 14 nicht aufrechterhalten wurde, kann eine vorher festgelegte Maßnahme eingeleitet werden, wie beispielsweise das Beenden der Behandlung, um einer Gefährdung des Patienten vorzubeugen. Hierzu ist in bislang nicht bekannter Art und Weise das Signal des Sensors 42 über die Leitung 46 an ein hier nicht näher dargestelltes Steuergerät weitergegeben worden, in dem nach Eintreffen des entsprechenden Signals geeignete Maßnahmen zur Beendigung des Behandlung getroffen wurden.

## Patentansprüche

1. Vorrichtung zur Behandlung einer medizinischen Flüssigkeit, welche eine Behandlungsmaschine (10) mit einer Ankoppelfläche (10) und einer auf der Ankoppelfläche (12) angeordneten flexiblen Matte (14) umfasst, wobei eine Kassette (18) aus einem Hartteil (20) mit flüssigkeitsführenden Kanälen, (24, 26, 28), welche von einer flexiblen Folie (22) abgedeckt sind, über die flexible Matte (14) an die Ankoppelfläche (12) der Behandlungsmaschine (10) ankoppelbar ist, wobei die Luft zwischen der flexiblen Folie (22) und der flexiblen Matte (14) über Absaugöffnungen in der Kassette (18) entfernbar ist,
**dadurch gekennzeichnet,**
**dass** eine Auslenkung der flexiblen Matte (14) oder von Teilen der flexiblen Matte (14) ermittelbar ist.

2. Vorrichtung nach Anspruch 1, **dadurch gekennzeichnet, dass** die flexible Matte (14) an vorbestimmten Stellen (36) flexibler ausgeführt ist, als in den übrigen Bereichen.

3. Vorrichtung nach Anspruch 1, **dadurch gekennzeichnet, dass** die flexible Matte (14) an vorbestimmten Stellen (36) dadurch flexibler ist, dass sie dünner als in den übrigen Bereichen ausgeführt ist.

4. Vorrichtung nach Anspruch 1, **dadurch gekennzeichnet, dass** die flexible Matte (14) an wenigstens einer vorbestimmten Stelle (36) mit einer Ringnut (14) umfasst wird.

5. Vorrichtung nach Anspruch 1, **dadurch gekennzeichnet, dass** die flexible Mat-te (14) an wenigstens einer vorbestimmten Stelle konkav oder konvex gegenüber den übrigen Stellen ausgeformt ist.

6. Vorrichtung gemäß Anspruch 1 oder 2, **dadurch gekennzeichnet, dass** die Überwachung der Auslenkung des flexibleren Bereichs (36) der flexiblen Matte durch einen Sensor (42) erfolgt.

7. Vorrichtung gemäß Anspruch 6, **dadurch gekennzeichnet, dass** der Sensor (42) ein Taster ist.

8. Vorrichtung gemäß Anspruch 6, **dadurch gekennzeichnet, dass** der Sensor (42) ein berührungslos arbeitender Sensor ist.

9. Vorrichtung gemäß Anspruch 8, **dadurch gekennzeichnet, dass** der Sensor (42) ein Ultraschallsensor, ein kapazitiver Sensor oder eine Lichtschranke ist.

10. Vorrichtung nach Anspruch 1, **dadurch gekennzeichnet, dass** als Sensor (42) ein Druck- oder Kraftsensor dient.

11. Verfahren zur Überprüfung der Dichtigkeit der Vorrichtung nach einem der Ansprüche 1 bis 10 nach dem Ankoppeln der medizinischen Kassette (18) an die Ankoppelfläche (12) der Behandlungsmaschine (10) über eine auf der Ankoppelfläche (12) angeordnete flexible Matte (14) und nach bzw. während des Absaugens von Luft zwischen der flexiblen Folie (22) und der flexiblen Matte (14) während des Ankoppelvorgangs und/oder bei angekoppelter Kassette (18),
**dadurch gekennzeichnet,**
**dass** das zwischen der flexiblen Folie (22) und der Ankoppelfläche (12) gebildete Vakuum durch einen Vergleich des Drucks zwischen der flexiblen Matte (14) und der Folie (22) einerseits mit dem Druck zwischen der flexiblen Matte (14) und der Ankoppelfläche (12) andererseits überprüft wird.

12. Vertahren nach Anspruch 11, **dadurch gekennzeichnet, dass** vor dem Ankoppeln der medizinischen Kassette (18) eine initiale Überprüfung der Messeinrichtung vor dem Ankoppeln der Kassette (18) durch Evakuieren des Zwischenraums zwischen Matte (14) und Ankoppelfläche (12) erfolgt.

13. Verfahren nach Anspruch 11 oder 12, **dadurch gekennzeichnet, dass** zur Bestimmung des Differenzdruckes die Auslenkung des der flexiblen Matte (14) oder von Teilen der flexiblen Matte (14) gemessen wird.

## Claims

1. An apparatus for the treatment of a medical liquid which includes a treatment machine (10) having a coupling surface (12) and a flexible mat (14) arranged on the coupling surface (12), wherein a cassette (18) made from a hard part (20) with liquid conducting passages (24, 26, 28) which are covered by a flexible film (22) can be coupled to the coupling surface (12) of the treatment machine (10) via the flexible mat (14), wherein the air between the flexile film (22) and the flexible mat (14) can be removed via suction openings in the cassette (18),
**characterized in that**
a deflection of the flexible mat (14) or of parts of the flexible mat (14) can be determined.

2. An apparatus in accordance with claim 1, **characterized in that** the flexible mat (14) is made more flexible at predetermined points (36) than in the other regions.

3. An apparatus in accordance with claim 1, **characterized in that** the flexible mat (14) is more flexible at predetermined points (36) **in that** it is made thinner than in the other regions.

4. An apparatus in accordance with claim 1, **characterized in that** the flexible mat (14) is encompassed by an annular groove (44) at at least one predetermined point (36).

5. An apparatus in accordance with claim 1, **characterized in that** the flexible mat (14) is made in concave form or in convex form at at least one predetermined point with respect to the other points.

6. An apparatus in accordance with either of claims 1 or 2, **characterized in that** the monitoring of the deflection of the more flexible region (36) of the flexible mat is carried out by a sensor (42).

7. An apparatus in accordance with claim 6, **characterized in that** the sensor (42) is a stylus.

8. An apparatus in accordance with claim 6, **characterized in that** the sensor (42) is a sensor working in a contactless manner.

9. An apparatus in accordance with claim 8, **characterized in that** the sensor (42) is an ultrasonic sensor, a capacitive sensor or a light barrier.

10. An apparatus in accordance with claim 1, **characterized in that** a pressure sensor or a force sensor serves as the sensor (42).

11. A method for the checking of the leak tightness of the apparatus in accordance with one of the claims 1 to 10 after the coupling of the medical cassette (18) to the coupling surface (12) of the treatment machine (10) via a flexible mat (14) arranged on the coupling surface (12) and after or during the suction of air between the flexible film (22) and the flexible mat (14) during the coupling process and/or with a coupled cassette (18), **characterized in that**
the vacuum formed between the flexible film (22) and the coupling surface (12) is checked by a comparison of the pressure between the flexible mat (14) and the film (22), on the one hand, with the pressure between the flexible mat (14) and the coupling surface (12), on the other hand.

12. A method in accordance with claim 11, **characterized in that**, before the coupling of the medical cassette (18), an initial check of the measurement device takes place before the coupling of the cassette (18) by evacuation of the intermediate space between the mat (14) and the coupling surface (12).

13. A method in accordance with either of claims 11 or 12, **characterized in that** the deflection of the flexible mat (14) or of parts of the flexible mat (14) is measured for the determination of the differential pressure.

## Revendications

1. Dispositif destiné au traitement d'un liquide médicinal, qui comprend une machine de traitement (10) avec une surface d'accouplement (12) et une natte flexible (14) disposée sur la surface d'accouplement (12), une cassette (18) étant accouplable à partir d'une pièce dure (20) avec des canaux conducteurs de liquide (24, 26, 28), qui sont recouverts d'un film flexible (22), via la natte flexible (14) à la surface d'accouplement (12) de la machine de traitement (10), l'air entre le film flexible (22) et la natte flexible (14) étant supprimable via des orifices d'aspiration dans la cassette (18),
**caractérisé**
**en ce qu'**une élongation de la natte flexible (14) ou de parties de la natte flexible (14) est calculable.

2. Dispositif selon la revendication 1, **caractérisé en ce que** la natte flexible (14) est réalisée de manière plus flexible dans des endroits prédéterminés (36) que dans les autres zones.

3. Dispositif selon la revendication 1, **caractérisé en ce que** la natte flexible (14) est plus flexible dans des endroits prédéterminés (36) du fait qu'elle est réalisée de manière plus fine que dans les autres zones.

4. Dispositif selon la revendication 1, **caractérisé en ce que** la natte flexible (14) est équipée dans au moins un endroit prédéterminé (36) d'une gorge de retenue (44).

5. Dispositif selon la revendication 1, **caractérisé en ce que** la natte flexible (14) est façonnée de manière concave ou convexe dans au moins un endroit prédéterminé par rapport aux autres endroits.

6. Dispositif selon les revendications 1 ou 2, **caractérisé en ce que** la surveillance de l'élongation de la zone plus flexible (36) de la natte flexible est réalisée par un capteur (42).

7. Dispositif selon la revendication 6, **caractérisé en ce que** le capteur (42) est un bouton.

8. Dispositif selon la revendication 6, **caractérisé en ce que** le capteur (42) est un capteur fonctionnant sans contact.

9. Dispositif selon la revendication 6, **caractérisé en ce que** le capteur (42) est un capteur à ultrasons, un capteur capacitif ou une barrière lumineuse.

10. Dispositif selon la revendication 6, **caractérisé en ce qu'**un capteur de pression ou d'énergie sert de capteur (42).

11. Procédé destiné à contrôler l'étanchéité du dispositif selon une quelconque des revendications 1 à 10 après l'accouplement de la cassette médicale (18) à la surface d'accouplement (12) de la machine de traitement (10) par l'intermédiaire d'une natte flexible (14) disposée sur la surface d'accouplement (12) et après ou pendant l'aspiration d'air entre le film flexible (22) et la natte flexible (14) pendant l'opération d'accouplement et/ou en cas de cassette accouplée (18),
**caractérisé en ce que**
le vide constitué entre le film flexible (22) et la surface d'accouplement (12) est contrôlé par une comparaison de la pression entre la natte flexible (14) et le film (22) avec la pression entre la natte flexible (14) d'une part et la surface d'accouplement (12) d'autre part.

12. Procédé selon la revendication 11, caractérisé entre que, avant l'accouplement de la cassette médicale (18), un contrôle initial du système de mesure s'effectue en amont de l'accouplement de la cassette (18) par évacuation de l'interstice entre la natte (14) et la surface d'accouplement.

13. Procédé selon les revendications 11 ou 12, caractérisé entre que, pour déterminer la pression différentielle, l'élongation de la natte flexible (14) ou de parties de la natte flexible (14) est mesurée.
